# EUROPÄISCHE PATENTSCHRIFT

(11) **EP 3 233 199 B1**
(45) Veröffentlichungstag und Bekanntmachung des Hinweises auf die Patenterteilung: **06.02.2019**
(21) Anmeldenummer: 15804760.5
(22) Anmeldetag: 03.12.2015
(51) Int. Cl.: A61K 8/26, A61K 8/23, A61K 8/89, A61Q 5/04

(54) **VERFAHREN ZUM FARBERHALT GEFÄRBTER UND/ODER AUFGEHELLTER KERATINISCHER FASERN**
METHOD FOR MAINTAINING THE COLOUR OF DYED AND/OR HIGHLIGHTED KERATIN FIBRES
PROCÉDÉ PERMETTANT LA TENUE DE LA COULEUR DE FIBRES KÉRATINIQUES COLORÉES ET/OU ÉCLAIRCIES

(30) Priorität: 17.12.2014 DE 102014226177
(43) Veröffentlichungstag der Anmeldung: 25.10.2017
(73) Patentinhaber: Henkel AG & Co. KGaA, 40589 Düsseldorf (DE)
(72) Erfinder: SCHULZE ZUR WIESCHE, Erik, 22453 Hamburg (DE); KROHN, René, 22851 Norderstedt (DE)
(86) Internationale Anmeldenummer: PCT/EP2015/078453
(87) Internationale Veröffentlichungsnummer: WO 2016/096449

(56) Entgegenhaltungen:
- DE-A1- 19 511 568
- US-A- 3 973 574
- US-B1- 6 200 374

## Beschreibung

Die Erfindung liegt auf dem Gebiet der Kosmetik und betrifft ein Verfahren zum Farberhalt gefärbter keratinischer Fasern, bei dem eine kosmetische Zusammensetzung vor der Haarfärbung und/oder Aufhellung als Vorbehandlungsmittel auf keratinische Fasern aufgebracht wird, wobei die kosmetische Zusammensetzung mindestens ein Alaun enthält und einen aciden pH-Wert aufweist. Die Erfindung betrifft weiterhin ein acides Vorbehandlungsmittel für Haare, das ein Alaun und ein Silikon enthält, sowie die Verwendung des Mittels zum Farberhalt gefärbter Haare.

Die Veränderung der Form und der Farbe von Haaren stellt einen wichtigen Bereich der modernen Kosmetik dar. Dadurch kann das Erscheinungsbild der Haare sowohl an aktuelle Modeströmungen als auch an die individuellen Wünsche des einzelnen Verbrauchers angepasst werden. Die modische Farbgestaltung von Frisuren oder die Kaschierung von ergrautem oder weißem Haar mit modischen oder natürlichen Farbtönen erfolgt üblicherweise mit farbverändernden Mitteln, welche die Haare permanent oder nur vorübergehend, d.h. temporär, färben.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus. Die Oxidationsfärbemittel zeichnen sich durch hervorragende, lang anhaltende Färbeergebnisse aus.

Für temporäre Färbungen werden üblicherweise Färbe- oder Tönungsmittel verwendet, welche als färbende Komponente sogenannte Direktzieher enthalten. Hierbei handelt es sich um Farbstoffmoleküle, welche direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Im Gegensatz zu den mit Oxidationsfärbemittel erhältliche Färbungen weise die Färbeergebnisse temporärer Färbungen eine geringere Haltbarkeit auf.

Die mit den zuvor beschriebenen Färbesystemen gefärbten keratinischen Fasern, insbesondere Haare, weisen jedoch den Nachteil auf, dass sie sich unter äußeren Einflüssen - beispielsweise während oder nach der Haarreinigung - in unerwünschter Weise verändern können.

Unter "unerwünschter Veränderung" wird das Verblassen oder Ausbluten, der Verlust der Farbbrillanz sowie die Farbverschiebung des durch die jeweilige Färbung erzielten Farbtons der Haare verstanden. Eine unerwünschte Veränderung der Haarfarbe tritt in der Regel während oder nach der Haarreinigung auf. Der Kontakt der Haare mit Wasser und Tensiden, aber auch das Einmassieren des Shampoos, das Abtrocknen der Haare nach dem Ausspülen des Shampoos oder die Fönhitze beim anschließenden Trocknungsvorgang können die Haftung der Haarfarbe beeinträchtigen und zu einer unerwünschten Farbveränderung und/oder zu weniger Brillanz der Haarfarbe führen. Durch weitere Umwelteinflüsse und/oder Sonneneinwirkungen kann die hervorgerufene unerwünschte Veränderung noch zusätzlich verstärkt werden.

In EP 1676604A1 wird ein Verfahren zur Verbesserung des Farbtons von Haaren beschrieben, bei dem die Haare zunächst mit einem Shampoo gewaschen werden, das neben einem anionischen Tensid und einem speziellen Silikon mindestens ein wasserlösliches Salz - bevorzugt Natriumsulfat - enthält. In einem zweiten Schritt werden die Haare mit einem Konditioniermittel, umfassend einen höheren Alkohol und ein kationisches Tensid im einem bestimmten Gewichtsverhältnis behandelt und anschließend ausgespült. In US 3 973 574 wird ein Haarfarbeverfahren offenbart, bei dem die Haare in einem ersten Schritt mit einer alkalischen Losung vorbehandelt und in einem zweiten Schritt mit einer wässrigen Mischung aus Kobaltchlorid und einem Alaun gebleicht werden.

In US 6 200 374 sind alaunhaltige Vorbehandlungsmittel offenbart, die im sauren pH Bereich liegen und für eine Anwendung rund um das menschliche Auge nicht geeignet sind.

In US 6 200 374 werden alaunsteinhaltige Losungen als Entwicklerkomponente in Haarfarbemitteln vorgeschlagen, welche natürliche Haarfarbstoffe wie Henna etc. enthalten.

Die im Stand der Technik bekannten Verfahren führen jedoch nicht immer zu dem gewünschten Farberhalt, insbesondere der Verringerung der Farbverschiebung.

Der vorliegenden Erfindung lag daher die Aufgabe zugrunde, ein kosmetisches Verfahren zur Behandlung keratinischer Fasern bereitzustellen, welches die Nachteile des Standes der Technik vermeidet bzw. zumindest abschwächt und welches zu einem verbesserten Farberhalt, insbesondere zu einer verminderten Farbverschiebung, führt. Das Verfahren soll einfach und schnell durchführbar sein und die behandelten Haare oder die Kopfhaut nicht (zusätzlich zur Färbung) belasten.

Es wurde nun überraschend gefunden, dass ein Verfahren, bei dem eine bestimmte kosmetische Zusammensetzung vor der Färbung und/oder Aufhellung (bzw. Bleichung) auf die keratinischen Fasern aufgebracht wird, zu einem verbesserten Farberhalt führt. Die kosmetische Zusammensetzung weist einen aciden pH-Wert auf und umfasst mindestens ein Alaun. Insbesondere die unerwünschte Verschiebung bzw. Veränderung der Haarfarbe in Richtung Gelb bzw. Blau kann durch das erfindungsgemäße Verfahren vermieden bzw. vermindert werden. Darüber hinaus gewährleistet das erfindungsgemäße Verfahren einen besonderen Haar - und/Kopfhautschutz. Die Haare und die Kopfhaut werden bei der Färbung weniger stark angegriffen, wodurch weniger Schädigungen (Rauigkeit, Spliss, Haarbruch) auftreten.

Ein erster Gegenstand der Erfindung ist daher ein kosmetisches Verfahren zum Farberhalt gefärbter keratinischer Fasern, bei dem
i. eine kosmetische Zusammensetzung als Vorbehandlungsmittel auf keratinische Fasern aufgebracht wird, und
ii. die keratinischen Fasern innerhalb eines Zeitraums von 5 Sekunden bis 24 Stunden nach Schritt i. einer Färbung und/oder Aufhellung unterzogen werden,
wobei die kosmetische Zusammensetzung - bezogen auf ihr Gewicht - 0,01 bis 4,00 Gew.-% mindestens eines Alauns enthält und einen pH-Wert im Bereich von 2,5 bis 5 aufweist.

Die kosmetische Zusammensetzung wird in einer bevorzugten Ausführungsform nicht ab- oder ausgespült, bevor Verfahrensschritt ii. durchgeführt wird.

Fakultativ können die Keratinfasern nach der Durchführung des Behandlungsschrittes i. getrocknet werden.

Unter keratinhaltigen bzw. keratinischen Fasern werden erfindungsgemäß Pelze, Wolle, Federn und insbesondere menschliche Haare verstanden.

Das erfindungsgemäße Verfahren ist prinzipiell auf keratinischen Fasern anwendbar, die mit permanenten, semipermanenten oder temporären Färbesystemen gefärbt werden und/oder
gebleicht
bzw. aufgehellt werden. Temporäre Färbesysteme sind jedoch dafür bestimmt, mit der Zeit ausgewaschen zu werden und/oder zu verblassen, deshalb ist das erfindungsgemäße Verfahren besonders geeignet für die Anwendung auf keratinischen Fasern, die mit permanenten oder oxidativen Haarfärbemitteln gefärbt werden und/oder die gebleicht werden.

In einer ersten bevorzugten Ausführungsform ist daher ein erfindungsgemäßes kosmetisches Verfahren bevorzugt, bei dem die keratinischen Fasern in Schritt ii. einer oxidativen Färbung unterzogen werden.

Es wurde gefunden, dass der durch das erfindungsgemäße Verfahren erzielbare Farberhalt durch diverse Faktoren weiter intensiviert werden kann. Zu diesen Faktoren zählen vor allem der pH-Wert der kosmetischen Zusammensetzung sowie die sorgfältige Auswahl besonders bevorzugter Alaune.

In einer zweiten bevorzugten Ausführungsform ist daher ein erfindungsgemäßes Verfahren besonders bevorzugt, bei dem die kosmetische Zusammensetzung einen pH-Wert im Bereich von 3,0 bis 4,9, besonders bevorzugt von 3,5 bis 4,8, und insbesondere bevorzugt von 4,0 bis 4,7 aufweist.

Vorbehandlungsmittel, die in diesem pH-Bereich formuliert werden, sind besonders mild, gut (kopf)hautverträglich und verleihen den Keratinfasern, insbesondere Haaren, einen besonderen Glanz.

Unter "Alaunen" sind bevorzugt Metallsulfatsalze - und/oder -doppelsalze der allgemeinen Formel M^{I}M^{III}(SO₄)₂ x 12H₂O zu verstehen, in der
- M^{I} bevorzugt für ein Alkalimetallion, ein Ammoniumion oder ein Guanidiniumion,
- M^{III} bevorzugt für ein Aluminium-, Gallium-, Indium-, Titan-, Vanadium-, Chrom-, Mangan-, Eisen-, Cobalt- oder Rhodiumion steht,
und die bevorzugt mit 12 Molekülen Kristallwasser kristallisieren.

Die für das erfindungsgemäße Verfahren besonders bevorzugten Alaune entsprechen der Formel M^{I}Al(SO₄)₂ x 12 H₂O, in der M' bevorzugt für ein Alkalimetallion, insbesondere für ein Natriumion oder ein Kaliumion, ein Ammoniumion oder ein Guanidiniumion stehen kann.

Insbesondere bevorzugt für den Farberhalt sind NaAl(SO₄)₂ x 12 H₂O, KAl(SO₄)₂ x 12 H₂O, NH₄Al(SO₄)₂ x 12 H₂O sowie Mischungen dieser Alaune.

In einer dritten bevorzugten Ausführungsform ist ein erfindungsgemäßes Verfahren dadurch gekennzeichnet, dass die kosmetische Zusammensetzung in Schritt i. ein Alaun der Formel M^{I}Al(SO₄)₂ x 12 H₂O enthält, in der M' für ein Kalium- Natrium-, Ammonium- und/oder ein Guanidiniumion steht.

In einer vierten besonders bevorzugten Ausführungsform ist ein erfindungsgemäßes Verfahren dadurch gekennzeichnet, dass die kosmetische Zusammensetzung in Schritt i. - bezogen auf ihr Gewicht - 0,05 bis 3,50 Gew.-%, bevorzugt 0,10 bis 3,00 Gew.-%, mehr bevorzugt 0,15 bis 2,50 Gew.-%, besonders bevorzugt 0,20 bis 2,25 Gew.-% und insbesondere bevorzugt 0,25 bis 2,00 Gew.-% mindestens eines Alauns, vorzugsweise eines Kalium-, Natrium-, Ammonium- oder eines Guanidiniumalauns enthält.

Die Eigenschaften der Haare und/oder der Kopfhaut können trotz Färbung und/oder Bleichung noch besser erhalten werden bzw. vor Schädigungen wie Rauigkeit, Spliss und/oder Haarbruch geschützt werden, wenn das erfindungsgemäße Verfahren mit kosmetischen Vorbehandlungsmitteln durchgeführt wird, die zusätzlich mindestens ein Silikon enthalten.

In einer fünften besonders bevorzugten Ausführungsform ist ein erfindungsgemäßes Verfahren demnach dadurch gekennzeichnet, dass die kosmetische Zusammensetzung in Schritt i. - bezogen auf ihr Gewicht - zusätzlich 0,001 bis 5,00 Gew.-% mindestens eines Silikons enthält.

Silikone bewirken auf dem Haar ausgezeichnete konditionierende Eigenschaften. Insbesondere bewirken sie eine bessere Kämmbarkeit der Haare in nassem und trockenem Zustand und wirken sich in vielen Fällen positiv auf den Haargriff und die Weichheit der Haare aus.

Geeignete Silikone, die in den kosmetischen Zusammensetzungen in Schritt i. des erfindungsgemäßen Verfahrens eingesetzt werden können, sind bevorzugt ausgewählt sein aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Die Verwendung spezieller Polyorganosiloxane (die im Folgenden beschrieben werden) in den kosmetischen Zusammensetzungen des erfindungsgemäßen Verfahrens führt jedoch zu einem besonders guten Farberhalt. Darüber hinaus steigert die zuvor genannte Verwendung die Geschmeidigkeit und Weichheit der keratinischen Fasern nach der Färbung und/oder Bleichung, ohne dass eine unerwünschte Beschwerung der Fasern auftritt.

Besonders geeignet sind Polyorganosiloxane der Formel (I) 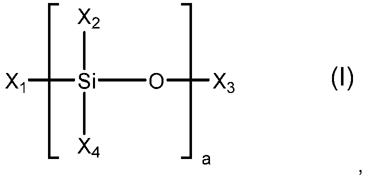 worin
X₁ und X₂ unabhängig voneinander OH, OR¹, R², O-PDMS oder O-fSiloxan bedeuten,
X₃ Wasserstoff oder einen einwertigen Kohlenwasserstoffrest mit 1 bis 8 Kohlenstoffatomen je Rest, PDMS oder fSiloxan bedeutet,
X₄ ein Rest der Formel
   -CH₂NHR⁴ , -CH₂NR⁴2 oder 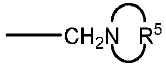
ist und
a eine Zahl von 1 bis 100, vorzugsweise eine Zahl von 1 bis 5, ist,
wobei
R¹ einen Alkylrest mit 1 bis 8 Kohlenstoffatomen bedeutet,
R² einen einwertigen, gegebenenfalls mit den Elementen N, P, S, O, Si und Halogen substituierten, gesättigten oder ungesättigten Kohlenwasserstoffrest mit 1 bis 200 Kohlenstoffatomen je Rest bedeutet,
PDMS für 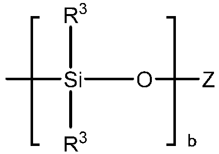 steht,
   fSiloxan für 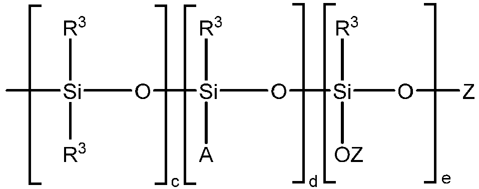 steht,

R³ unabhängig voneinander jeweils einen einwertigen, gegebenenfalls mit den Elementen N, P, S, O, Si und Halogen substituierten, gesättigten oder ungesättigten Kohlenwasserstoffrest mit 1 bis 200 Kohlenstoffatomen je Rest bedeutet,
A ein Rest der Formel R⁶-[NR⁷-_{R}⁸-]_{f}NR⁷₂ bedeutet,
   wobei
R⁶ ein zweiwertiger linearer oder verzweigter Kohlenwasserstoffrest mit 3 bis 18 Kohlenstoffatomen bedeutet,
R⁷ ein Wasserstoffatom, einen Alkylrest mit 1 bis 8 Kohlenstoffatomen oder einen Acylrest bedeutet,
R⁸ ein zweiwertiger Kohlenwasserstoffrest mit 1 bis 6 Kohlenstoffatomen bedeutet,
b eine Zahl von 1 bis 2000, vorzugsweise von 1 bis 1000, ist,
c 0 oder eine Zahl von 1 bis 2000, vorzugsweise von 50 bis 1000, ist,
d eine Zahl von 1 bis 1000, vorzugsweise von 1 bis 10, ist,
e 0 oder eine Zahl von 1 bis 5 ist,
f 0, 1, 2, 3 oder 4 ist,
Z Wasserstoff, einen Alkylrest mit 1 bis 8 Kohlenstoffatomen oder 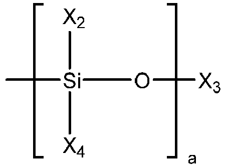 bedeutet,

R⁴ einen einwertigen, gegebenenfalls N- und/oder O-Atome enthaltenden Kohlenwasserstoffrest mit 1 bis 18 Kohlenstoffatomen bedeutet und
R⁵ einen zweiwertigen gegebenenfalls N- und/oder O-Atome enthaltenden Kohlenwasserstoffrest mit 3 bis 12 Kohlenstoffatomen bedeutet.

Beispiele für einen Alkylrest R¹ sind Methyl-, Ethyl-, *n*-Propyl-, *iso*-Propyl-, 1-*n*-Butyl-, 2-*n*-Butyl-, *iso-*Butyl-, *tert*.-Butyl-, *n*-Pentyl-, *iso*-Pentyl-, *neo*-Pentyl-, *tert*.-Pentyl, *n*-Hexyl, *n*-Heptyl, *n*-Octyl, *iso-*Octyl oder 2,2,4-Trimethylpentyl-, wobei Methyl-, Ethyl- und Butyl- bevorzugt sind.

Beispiele für Kohlenwasserstoffreste R² und R³ sind Alkylreste, wie Methyl-, Ethyl-, *n*-Propyl-, *iso-*Propyl-, 1-*n*-Butyl-, 2-*n*-Butyl-, *iso*-Butyl-, *tert*.-Butyl-, *n*-Pentyl-, *iso*-Pentyl-, *neo*-Pentyl-, *tert*.-Pentyl, *n*-Hexyl, *n*-Heptyl, *n*-Octyl, *iso*-Octyl, 2,2,4-Trimethylpentyl-, *n*-Nonyl-, *n*-Decyl-, *n*-Dodecyl-, *n-*Octadecyl-, Cyclopentyl-, Cyclohexyl-, Cycloheptyl-, Methylcyclohexyl-, Vinyl-, 5-Hexenyl-, Cyclohexenyl-, 1-Propenyl-, Allyl-, 3-Butenyl-, 4-Pentenyl-, Phenyl-, Naphthyl-, Anthryl-, Phenanthryl-, o-Tolyl-, m-Tolyl, p-Tolyl-, Xylyl-, Ethylphenyl-, Benzyl-, alpha-Phenylethyl- und beta-Phenylethylreste. Bevorzugt sind als Rest R² der Methyl-, Ethyl-, Octyl- und Phenylrest, besonders bevorzugt sind der Methyl- und Ethylrest.

Beispiele für halogenierte Reste R² und R³ sind der 3,3,3-Trifluor-n-propyl-, 2,2,2,2',2',2'-Hexafluorisopropyl-, Heptafluorisopropyl-, o-Chlorphenyl-, m-Chlorphenyl und p-Chlorphenylrest.

Beispiele für R⁴ sind die für die Kohlenwasserstoffreste R² und R³ aufgeführten Alkyl-, Cycloalkyl-, Aryl-, Alkaryl- und Aralkylreste.

Bevorzugte Beispiele für R⁵ sind Reste der Formeln -CH₂-CH₂-O-CH₂-CH₂-, -CH₂-CH₂-NH-CH₂-CH₂- oder -CH₂-CH₂-NH-CH₂-, wobei der Rest -CH₂-CH₂-O-CH₂-CH₂- besonders bevorzugt ist.

Beispiele für R⁶ sind Alkylenrest mit 3 bis 10 Kohlenstofftomen wie Propylen, Butylen, Pentylen, Hexylen, Heptylen, Octylen, Nonylen und Decylen.

R⁷ kann ein Wasserstoffatom, ein Methyl-, Ethyl-, *n*-Propyl-, *iso*-Propyl-, 1-*n*-Butyl-, 2-*n*-Butyl-, *iso-*Butyl-, *tert*.-Butyl-, *n*-Pentyl-, *iso*-Pentyl-, *neo*-Pentyl-, *tert*.-Pentyl, *n*-Hexyl, *n*-Heptyl, *n*-Octyl, *iso-*Octyl, 2,2,4-Trimethylpentyl- oder Acetylrest sein, wobei ein Wasserstoffatom bevorzugt ist.

Bevorzugte Beispiele für R⁸ sind Alkylenreste wie Methylen, Ethylen, Propylen, Butylen, Pentylen, oder Hexylen.

Z ist bevorzugt Wasserstoff oder Methyl-, Ethyl-, *n*-Propyl-, *iso*-Propyl-, 1-*n*-Butyl-, 2-*n*-Butyl-, *iso-*Butyl-, *tert*.-Butyl-, *n*-Pentyl-, *iso*-Pentyl-, *neo*-Pentyl-, *tert*.-Pentyl, *n*-Hexyl, *n*-Heptyl, *n*-Octyl, *iso-*Octyl oder 2,2,4-Trimethylpentyl-, wobei Wasserstoff, Methyl-, Ethyl- und Butyl- besonders bevorzugt sind.

Bevorzugte Reste X₄ sind entsprechend der obigen Definitionen für R⁴ und R⁵ Aminomethyl-, Methylaminomethyl-, Dimethylaminomethyl-, Diethylaminomethyl-, Dibutylaminomethyl-, Cyclohexylaminomethyl-, Morpholinomethyl-, Piperidinomethyl-, Piperazinomethyl-, ((Diethoxymethylsilyl)methyl)cyclohexylaminomethyl-, ((Triethoxysilyl)methyl)cyclohexylaminomethyl-, Anilinomethyl-, 3-Dimethylaminopropylaminomethyl-, Bis(3-dimethylaminopropyl)aminomethylrest und Mischungen daraus. Dabei ist es höchst bevorzugt, wenn das Haarbehandlungsmittel Polyorganosiloxane der Formel (I) enthält, die als Rest X₄ den Morpholinomethylrest enthalten.

Entsprechend der Definitionen für R⁶, R⁷ und R⁸ sind bevorzugte Beispiele für Rest A:

-(CH2)3NH2

-(CH2)3-NH-(CH2)2-NH2

-CH₂CH(CH₃)CH₂-NH-(CH₂)₂-NH₂

-(CH₂)₃-NH(Cyclohexyl)

-(CH₂)₃-NHCH₃

-(CH2)3-N(CH3)2

-(CH₂)₃-NHCH₂CH₃

-(CH2)₃-N(CH₂CH₃)₂

-(CH2)4-NH2

-CH₂CH(CH₃)CH₂-NH₂

-(CH₂)₃-NH-(CH₂)₂-NHCH₃

-(CH2)₃-NH-(CH₂)₂-N(CH₃)₂

-(CH₂)₃-NH-(CH₂)₂-NHCH₂CH₃

-(CH₂)₃-NH-(CH₂)₂-N(CH₂CH₃)₂

-(CH₂)₃[-NH-CH₂CH₂]₂-NH₂

-(CH₂)₃-NH(Acetyl)

-(CH₂)₃-NH-(CH₂)₂-NH(Acetyl) und

-(CH₂)₃-N(Acetyl)-(CH₂)₂-NH(Acetyl).

Zur Herstellung der Polyorganosiloxane der Formel (I) werden vorzugsweise handelsübliche Polydimethylsiloxane mit endständigen Silanolgruppen und/oder Polydimethylsiloxane mit endständigen Alkoxy- und Silanolgruppen und/oder Amin-funktionalisierte Siloxane, die Silanolgruppen oder Alkoxy- und Silanolgruppen enthalten, mit einem Dialkoxy- und/oder Trialkoxysilan, welches einen Rest Formel
-CH₂NHR⁴, -CH₂NR⁴₂ oder 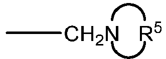 aufweist, umgesetzt.

Entsprechend steht in Formel (I) "fSiloxan" für einen Rest, der sich von einem Amin-funktionalisierten Siloxan ableitet.

Besonders bevorzugt werden Trialkoxysilane oder eine Mischung von Dialkoxy- und Trialkoxysilanen, eingesetzt, wobei der Einsatz von Trialkoxysilanen alleine besonders bevorzugt ist. Beim Einsatz von Trialkoxysilanen oder einer Mischung von Dialkoxy- und Trialkoxysilanen werden unabhängig von der Struktur der eingesetzten Siloxane und der Position der Alkoxy- und/oder Silanolgruppen in den Siloxanen zumindest teilweise vernetzte Polyorganopolysiloxane erhalten. In einer ganz besonders bevorzugten Ausführungsform enthält das kosmetische Mittel vernetzte Polyorganosiloxane. In einer höchst bevorzugten Ausführungsform enthält das kosmetische Mittel vernetzte Polyorganosiloxane, die aus der Umsetzung von Siloxanen und Trialkoxysilanen hervorgegangen sind.

Bevorzugte Beispiele für die eingesetzten Dialkoxy- oder Trialkoxysilane umfassen:
Diethylaminomethylmethyldimethoxysilan,
Dibutylaminomethyltriethoxysilan,
Dibutylaminomethyltributoxysilan,
Cyclohexylaminomethyltrimethoxysilan,
Cyclohexylaminomethyltriethoxysilan,
Cyclohexylaminomethyl-methyldiethoxysilan,
Anilinomethyltriethoxysilan,
Anilinomethylmethyldiethoxysilan,
Morpholinomethyltriethoxysilan,
Morpholinomethyltrimethoxysilan,
Morpholinomethyltriisopropoxysilan,
3-Dimethylaminopropyl-aminomethyltrimethoxysilan,
Morpholinomethyltributoxysilan,
Morpholinomethyltrialkoxysilan, wobei der Alkoxyrest ein C₁-C₄-Alkoxyrest ist, insbesondere ein Gemisch aus Methoxy- und Ethoxyrest ist,
Piperazinomethyltriethoxysilan,
Piperidinomethyltriethoxysilan und
Teilhydrolysate davon.

Ein besonders bevorzugtes Silan ist Morpholinomethyltriethoxysilan.

Ein besonders bevorzugt einsetzbares Amin-funktionalisiertes Siloxan ist ein Copolymer aus 3-(2-Aminoethylamino)propylmethylsiloxy- und Dimethylsiloxyeinheiten, welches Silanolgruppen oder Alkoxy- und Silanolgruppen aufweist.

Bei der Herstellung der Polyorganosiloxane der Formel (I) können gleiche oder verschiedene Siloxane sowie gleiche oder verschiedene Silane eingesetzt werden.

Ein insbesondere bevorzugtes Polyorganosiloxan ist beispielsweise unter der INCI-Bezeichnung Amodimethicone/Morpholinomethyl Silsesquioxane Copolymer und/oder unter der CAS-Nummer 1293390-78-9 bekannt. Kommerziell erhältlich ist dieses Polyorganosiloxan unter der Bezeichnung Belsil® ADM 8301 E (ex Wacker). Der Rohstoff stellt eine Mikroemulsion dar und weist die folgenden Bestandteile auf: Amodimethicone/Morpholinomethyl Silsesquioxane Copolymer, Trideceth-5, Glycerin, Phenoxyethanol und Wasser.

Der Einsatz der zuvor beschriebenen Polyorganosiloxane erfolgt vorzugsweise als wässrige Suspensionen oder wässrige Emulsionen von Polyorganosiloxanen. Die Dispersionen können ein oder mehrere Tenside als Dispergatoren enthalten. Die Tenside können von beliebiger Art sein, ionisch und / oder nichtionisch. Alternativ können anorganische Feststoffe wie Kieselsäuren und/oder Bentonite als Dispergatoren eingesetzt sein. Die mittels Lichtstreuung in den Dispersionen gemessene mittlere Teilchengrösse der Polyorganosiloxane liegt vorzugsweise im Bereich 0,001 bis 100 µm, mehr bevorzugt bei 0,002 bis 10 µm. Die pH-Werte können von 1 bis 14 variieren. Bevorzugt liegt der pH-Wert von 3 bis 9, besonders bevorzugt von 5 bis 8.

Innerhalb der fünften bevorzugten Ausführungsform ist ein erfindungsgemäßes Verfahren besonders bevorzugt, bei dem als Silikon mindestens eine unter der INCI-Bezeichnung Amodimethicone/Morpholinomethyl Silsesquioxane Copolymer bekannte Verbindung eingesetzt wird.

Das Vorbehandlungsmittel, welches in Verfahrensschritt i. des erfindungsgemäßen Verfahrens angewendet wird, kann als niedrig-viskose Wasser-basierte Emulsion, als Spray, als Creme, Gel, Lotion, Paste, Shampoo oder Conditioner konfektioniert sein.

Das erfindungsgemäße Verfahren umfasst das Aufbringen des Vorbehandlungsmittels auf keratinische Fasern und eine sich innerhalb eines Zeitraumes von 5 Sekunden bis 24 Stunden daran anschließenden oxidative Färbe- und/oder Bleichbehandlung.

Ein großer Vorteil der in Schritt i. eingesetzten Vorbehandlungsmittel ist, dass sie nicht nur dann wirksam sind, wenn sie unmittelbar vor der oxidativen Färbebehandlung angewendet werden, sondern bis zu 24 Stunden vorher angewendet werden können, ohne dass durch äußere Einflüsse eine Abschwächung der Wirkung zu befürchten wäre. Auf diese Weise ist es möglich, beispielsweise morgens nach der Haarwäsche Schritt i. des erfindungsgemäßen Verfahrens durchzuführen und die oxidative Färbebehandlung erst abends.

Erfindungsgemäß bevorzugte Verfahren sind dadurch gekennzeichnet, dass der Zeitraum zwischen den Verfahrensschritten i. und ii. von 5 Sekunden bis 20 Minuten, bevorzugt 30 Sekunden bis 10 Minuten, besonders bevorzugt 1 bis 5 Minuten, beträgt.

Weitere erfindungsgemäß bevorzugte Verfahren sind dadurch gekennzeichnet, dass man das in Verfahrensschritt i. applizierte Vorbehandlungsmittel für einen Zeitraum von 5 Sekunden bis 120 Minuten, bevorzugt 10 Sekunden bis 10 Minuten, auf das Haar einwirken lässt, bevor mit Verfahrensschritt ii. begonnen wird.

Weitere erfindungsgemäß bevorzugte Verfahren sind dadurch gekennzeichnet, dass man das in Verfahrensschritt i. applizierte Vorbehandlungsmittel für einen Zeitraum von 5 Sekunden bis 120 Minuten, bevorzugt 10 Sekunden bis 10 Minuten, auf das Haar einwirken lässt, bevor Verfahrensschritt ii. erfolgt.

Das erfindungsgemäße Verfahren ist in einer weiteren bevorzugten Ausführungsform dadurch gekennzeichnet, dass Schritt ii. das Aufbringen eines Haarfärbemittels umfasst, welches vorzugsweise mindestens ein Oxidationsfarbstoffvorprodukt vom Entwicklertyp und mindestens ein Oxidationsfarbstoffvorprodukt vom Kupplertyp sowie mindestens ein Oxidationsmittel enthält.

Bevorzugte Oxidationsmittel sind ausgewählt aus Peroxoverbindungen, bevorzugt ausgewählt aus Wasserstoffperoxid, festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon·n H₂O₂ (n ist eine positive ganze Zahl größer 0), Harnstoffperoxid und Melaminperoxid, weiterhin ausgewählt aus Diammoniumperoxodisulfat (auch als Ammoniumpersulfat bezeichnet), Dinatriumperoxodisulfat (auch als Natriumpersulfat bezeichnet) und Dikaliumperoxodisulfat (auch als Kaliumpersulfat bezeichnet) sowie aus Mischungen dieser Oxidationsmittel. Erfindungsgemäß ganz besonders bevorzugte Oxidationsmittel sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-Gewichtsprozentige Lösungen in Wasser verwendet. Erfindungsgemäß bevorzugte Verfahren sind dadurch gekennzeichnet, dass die in Schritt ii. eingesetzten Haarfärbemittel - bezogen auf ihr Gewicht - 0,5 bis 12 Gew.-%, bevorzugt 2 bis 10 Gew.-%, besonders bevorzugt 3 bis 6 Gew.-% Wasserstoffperoxid (berechnet als 100%iges H₂O₂) enthalten.

Oxidative Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des in Schritt ii. eingesetzten Haarfärbemittels im Bereich von 7 und 11, insbesondere im Bereich von 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes verwendbaren Alkalisierungsmittel können aus der Gruppe Ammoniak, basischen Aminosäuren, Alkalihydroxiden, Alkanolaminen, Alkalimetallmetasilikaten, Alkaliphosphaten und Alkalihydrogenphosphaten, ausgewählt werden. Als Alkalimetallionen dienen bevorzugt Lithium, Natrium, Kalium, insbesondere Natrium oder Kalium. Die als Alkalisierungsmittel einsetzbaren basischen Aminosäuren werden bevorzugt ausgewählt aus der Gruppe L-Arginin, D-Arginin, D,L-Arginin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als Alkalisierungsmittel einsetzbaren Alkalihydroxide werden bevorzugt ausgewählt aus der Gruppe Natriumhydroxid und Kaliumhydroxid.

Die als Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine sind ausgewählt aus der Gruppe 2-Aminoethan-1-ol, 2-Amino-2-methylpropan-1-ol und 2-Amino-2-methyl-propan-1,3-diol.

Für eine Färbung, die eine starke Aufhellung sehr dunklen Haares erfordert, ist der Einsatz von Wasserstoffperoxid oder dessen Anlagerungsprodukten an organische bzw. anorganische Verbindungen oftmals nicht ausreichend. In diesen Fällen wird in der Regel eine Kombination aus Wasserstoffperoxid und Peroxodisulfatsalzen (Persulfatsalzen) eingesetzt. Bevorzugte Persulfatsalze sind Ammoniumperoxidisulfat, Kaliumperoxidisulfat, Natriumperoxidisulfat, sowie Mischungen hiervon.

Das mindestens eine Persulfatsalz ist bevorzugt in einer Gesamtmenge von 0,1 bis 25 Gew.-%, besonders bevorzugt in einer Gesamtmenge von 1 bis 15 Gew.-%, bezogen auf das Gewicht des anwendungsbereiten Färbemittels, enthalten.

Oxidationsfarbstoffvorprodukte können aufgrund ihres Reaktionsverhaltens in zwei Kategorien eingeteilt werden, so genannte Entwicklerkomponenten und Kupplerkomponenten. Kupplerkomponenten bilden im Rahmen der oxidativen Färbung allein keine signifikante Färbung aus, sondern benötigen stets die Gegenwart von Entwicklerkomponenten. Entwicklerkomponenten können mit sich selbst den eigentlichen Farbstoff ausbilden.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride oder Hydrobromide oder der Sulfate einzusetzen.

Überraschend wurde festgestellt, dass mit dem erfindungsgemäßen Verfahren unter Verwendung von mindestens einem Oxidationsfarbstoffvorprodukt vom Entwicklertyp und mindestens einem Oxidationsfarbstoffvorprodukt vom Kupplertyp Haarfärbungen mit besonders hoher Waschechtheit erzielt werden konnten. Eine besonders gute Verbesserung der Waschechtheit wurde für Rezepturen mit der Entwickler/Kuppler-Kombination 1-Hydroxyethyl-4,5-diaminopyrazol/3-Aminophenol beobachtet. Auch die Reduzierung der Haarschädigung war überraschend hoch.

Besonders bevorzugte Entwicklerkomponenten sind ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxy-ethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, den physiologisch verträglichen Salzen dieser Verbindungen sowie Mischungen dieser Entwicklerkomponenten und Entwicklerkomponentensalze.

Ganz besonders bevorzugte Entwicklerkomponenten sind ausgewählt aus 4,5-Diamino-1-(2-hydroxyethyl)pyrazol, p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin und Mischungen dieser Verbindungen sowie deren physiologisch verträglichen Salzen. Außerordentlich bevorzugt ist 4,5-Diamino-1-(2-hydroxyethyl)pyrazol und dessen physiologisch verträglichen Salze.

Die Entwicklerkomponenten werden bevorzugt in einer Gesamtmenge von 0,005 bis 10 Gew.-%, besonders bevorzugt 0,1 bis 5 Gew.-%, jeweils bezogen auf das Gewicht des anwendungsbereiten Färbemittels, verwendet.

Unter dem Begriff "anwendungsbereites Färbemittel" wird im Sinne dieser Anmeldung die Mischung aus sämtlichen Oxidationsfarbstoffvorprodukten und sämtlichen Oxidationsmitteln, ggf. in Kombination mit einem geeigneten kosmetischen Träger, z.B. einer Cremebasis, sowie ggf. in Kombination mit mindestens einem direktziehenden Farbstoff, verstanden.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt cyclische Verbindungen, die am Cyclus mindestens zwei Gruppen tragen, ausgewählt aus (i) gegebenenfalls substituierten Aminogruppen und/oder (ii) Hydroxylgruppen. Wenn die cyclische Verbindung ein Sechsring (bevorzugt aromatisch) ist, so befinden sich die besagten Gruppen bevorzugt in ortho-Position oder meta-Position zueinander.

Bevorzugte erfindungsgemäße Verfahren sind dadurch gekennzeichnet, dass das mindestens eine Oxidationsfarbstoffvorprodukt vom Kupplertyp ausgewählt ist aus einer der folgenden Klassen:
- 3-Aminophenol (m-Aminophenol) und/oder dessen Derivate,
- 3-Aminoanilin (m-Diaminobenzol) und/oder dessen Derivate,
- 2-Aminoanilin (1,2-Diaminobenzol; o-Diaminobenzol) und/oder dessen Derivate,
- 2-Aminophenol (o-Aminophenol) und/oder dessen Derivate,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin,
Gemische aus zwei oder mehreren Verbindungen aus einer oder mehreren dieser Klassen sind im Rahmen dieser Ausführungsform ebenso erfindungsgemäß bevorzugt.

Erfindungsgemäß besonders bevorzugte zusätzliche Kupplerkomponenten sind ausgewählt aus 3-Aminophenol, 5-Amino-2-methylphenol, 3-Amino-2-chlor-6-methylphenol, 2-Hydroxy-4-amino-phenoxyethanol, 5-Amino-4-chlor-2-methylphenol, 5-(2-Hydroxyethyl)amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol (= 2-Amino-4-Hydroxyethylaminoanisol), 1,3-Bis (2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxy-pyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen. Ganz besonders bevorzugt sind dabei 3-Aminophenol, Resorcin, 2-Methylresorcin, 5-Amino-2-methylphenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie deren physiologisch verträglichen Salze und Mischungen der genannten Komponenten.

Die mindestens eine Kupplerkomponente wird bevorzugt in einer Gesamtmenge von 0,005 bis 20 Gew.-%, vorzugsweise 0,1 bis 5 Gew.-%, jeweils bezogen auf das Gewicht des anwendungsbereiten Oxidationsfärbemittels, verwendet.

Im Rahmen der vorliegenden Erfindung sind folgende Kombinationen aus Oxidationsfarbstoffvorprodukten vom Entwicklertyp und vom Kupplertyp besonders bevorzugt, wobei die Aminverbindungen und die Stickstoffheterocyclen auch in Form ihrer physiologisch verträglichen Salze vorliegen können:
p-Toluylendiamin / Resorcin;
p-Toluylendiamin / 2-Methylresorcin;
p-Toluylendiamin / 5-Amino-2-methylphenol;
p-Toluylendiamin / 3-Aminophenol;
p-Toluylendiamin / 2-(2,4-Diaminophenoxy)ethanol;
p-Toluylendiamin / 1,3-Bis(2,4-diaminophenoxy)propan;
p-Toluylendiamin / 1 -Methoxy-2-amino-4-(2-hydroxyethylamino)benzol;
p-Toluylendiamin / 2-Amino-3-hydroxypyridin;
p-Toluylendiamin / 1-Naphthol;
2-(2-Hydroxyethyl)-p-phenylendiamin / Resorcin;
2-(2-Hydroxyethyl)-p-phenylendiamin / 2-Methylresorcin;
2-(2-Hydroxyethyl)-p-phenylendiamin / 5-Amino-2-methylphenol;
2-(2-Hydroxyethyl)-p-phenylendiamin / 3-Aminophenol;
2-(2-Hydroxyethyl)-p-phenylendiamin / 2-(2,4-Diaminophenoxy)ethanol;
2-(2-Hydroxyethyl)-p-phenylendiamin / 1,3-Bis(2,4-diaminophenoxy)propan;
2-(2-Hydroxyethyl)-p-phenylendiamin / 1-Methoxy-2-amino-4-(2-hydroxyethylamino)benzol;
2-(2-Hydroxyethyl)-p-phenylendiamin / 2-Amino-3-hydroxypyridin;
2-(2-Hydroxyethyl)-p-phenylendiamin / 1-Naphthol;
2-Methoxymethyl-p-phenylendiamin / Resorcin;
2-Methoxymethyl-p-phenylendiamin / 2-Methylresorcin;
2-Methoxymethyl-p-phenylendiamin / 5-Amino-2-methylphenol;
2-Methoxymethyl-p-phenylendiamin / 3-Aminophenol;
2-Methoxymethyl-p-phenylendiamin / 2-(2,4-Diaminophenoxy)ethanol;
2-Methoxymethyl-p-phenylendiamin / 1,3-Bis(2,4-diaminophenoxy)propan;
2-Methoxymethyl-p-phenylendiamin / 1-Methoxy-2-amino-4-(2-hydroxyethylamino)benzol;
2-Methoxymethyl-p-phenylendiamin / 2-Amino-3-hydroxypyridin;
2-Methoxymethyl-p-phenylendiamin / 1-Naphthol;
N-(4-Amino-3-methylphenyl)-N-[3-(1H-imidazol-1-yl)propyl]amin / Resorcin;
N-(4-Amino-3-methylphenyl)-N-[3-(1H-imidazol-1-yl)propyl]amin / 2-Methylresorcin;
N-(4-Amino-3-methylphenyl)-N-[3-(1H-imidazol-1-yl)propyl]amin / 5-Amino-2-methylphenol;
N-(4-Amino-3-methylphenyl)-N-[3-(1H-imidazol-1-yl)propyl]amin / 3-Aminophenol;
N-(4-Amino-3-methylphenyl)-N-[3-(1H-imidazol-1-yl)propyl]amin / 2-(2,4-Diaminophenoxy)ethanol;
N-(4-Amino-3-methylphenyl)-N-[3-(1H-imidazol-1-yl)propyl]amin / 1,3-Bis(2,4-diaminophenoxy)-propan;
N-(4-Amino-3-methylphenyl)-N-[3-(1H-imidazol-1-yl)propyl]amin / 1-Methoxy-2-amino-4-(2-hydroxy-ethylamino)benzol;
N-(4-Amino-3-methylphenyl)-N-[3-(1H-imidazol-1-yl)propyl]amin / 2-Amino-3-hydroxypyridin; N-(4-Amino-3-methylphenyl)-N-[3-(1H-imidazol-1-yl)propyl]amin / 1-Naphthol; 4,5-Diamino-1-(2-hydroxyethyl)pyrazol / Resorcin;
4,5-Diamino-1-(2-hydroxyethyl)pyrazol / 2-Methylresorcin;
4,5-Diamino-1-(2-hydroxyethyl)pyrazol / 5-Amino-2-methylphenol;
4,5-Diamino-1-(2-hydroxyethyl)pyrazol / 3-Aminophenol;
4,5-Diamino-1-(2-hydroxyethyl)pyrazol / 2-(2,4-Diaminophenoxy)ethanol;
4,5-Diamino-1-(2-hydroxyethyl)pyrazol / 1,3-Bis(2,4-diaminophenoxy)propan;
4,5-Diamino-1-(2-hydroxyethyl)pyrazol / 1-Methoxy-2-amino-4-(2-hydroxyethylamino)benzol;
4,5-Diamino-1-(2-hydroxyethyl)pyrazol / 2-Amino-3-hydroxypyridin;
4,5-Diamino-1-(2-hydroxyethyl)pyrazol / 1-Naphthol.

Erfindungsgemäß besonders bevorzugt sind die Kombinationen 4,5-Diamino-1-(2-hydroxyethyl)-pyrazol/3-Aminophenol und p-Toluylendiamin/3-Aminophenol. Außerordentlich bevorzugt, besonders im Hinblick auf die Verbesserung der Waschechtheit, ist die Kombination 4,5-Diamino-1-(2-hydroxyethyl)pyrazol/3-Aminophenol.

Um eine ausgewogene und subtile Nuancenausbildung zu erzielen, ist es erfindungsgemäß bevorzugt, wenn weitere farbgebende Komponenten in dem Färbemittel enthalten sind, das in Schritt ii. des erfindungsgemäßen Verfahrens eingesetzt wird.

In einer weiteren Ausführungsform können die in Schritt ii. dieser Variante des erfindungsgemäßen Verfahrens eingesetzten Mittel zusätzlich mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Ein weiteres bevorzugtes erfindungsgemäßes Verfahren ist dadurch gekennzeichnet, dass das in Schritt ii. applizierte Färbemittel nach einer Zeit von 5-60 Minuten, bevorzugt 30 - 45 Minuten, von den keratinischen Fasern abgespült wird.

Das im erfindungsgemäßen Verfahren in Schritt ii. eingesetzte Färbemittel wird bevorzugt aus einem Zweikomponentenmittel hergestellt, wobei eine Komponente die Oxidationsfarbstoffvorprodukte und die andere Komponenten das oder die Oxidationsmittel enthält. Das anwendungsbereite Färbemittel für Schritt ii. wird dann durch Mischen beider Komponenten direkt vor dem Applikationsschritt ii. hergestellt. Eine Auftrennung in Mehrkomponentensysteme bietet sich insbesondere dort an, wo Inkompatibilitäten der Inhaltsstoffe zu erwarten oder zu befürchten sind.

Ein zweiter Gegenstand der Erfindung ist ein kosmetisches Mittel zur Vorbehandlung keratinischer Fasern vor einer (vorzugsweise oxidativen) Färbung und/oder Aufhellung, das - bezogen auf sein Gewicht -
a) 0,01 bis 4,00 Gew.-% mindestens eines Alauns und
b) 0,001 bis 5 Gew.-% mindestens eines Silikons enthält, und
einen pH-Wert im Bereich von 2,5 bis 5 aufweist.

Für bevorzugte Ausführungsformen des erfindungsgemäßen kosmetischen Mittels, insbesondere hinsichtlich bevorzugter Ausführungsformen des Silikons und des Alauns, gilt mutatis mutandis das zu dem erfindungsgemäßen Verfahren Gesagte.

In einer ersten bevorzugten Ausführungsform enthalten die kosmetischen Vorbehandlungsmittel des zweiten Erfindungsgegenstandes - bezogen auf ihr Gewicht - bevorzugt 75,00 bis 99,98 Gew.-%, mehr bevorzugt 80,00 bis 99,50 Gew.-%, besonders bevorzugt 85,00 bis 99,00 Gew.-% und insbesondere 90,00 bis 98,50 Gew.-% Wasser.

Erfindungsgemäß besonders bevorzugte Vorbehandlungsmittel des zweiten Erfindungsgegenstandes sind dadurch gekennzeichnet, dass sie als Silikon mindestens eine unter der INCI-Bezeichnung Amodimethicone/Morpholinomethyl Silsesquioxane Copolymer bekannte Verbindung in einer Gesamtmenge von 0,001 bis 5,00 Gew.-%, bevorzugt 0,005 bis 4,00 Gew.-%, besonders bevorzugt 0,01 - 2,00 Gew.-%, außerordentlich bevorzugt 0,02 - 1,00 Gew.-%, jeweils bezogen auf das Gesamtgewicht des Vorbehandlungsmittels, enthalten.

Insbesondere bevorzugt sind kosmetische Vorbehandlungsmittel des zweiten Erfindungsgegenstandes, die - bezogen auf ihr Gewicht -
a) 0,05 bis 3,50 Gew.-%, bevorzugt 0,10 bis 3,00 Gew.-%, mehr bevorzugt 0,15 bis 2,50 Gew.-%, besonders bevorzugt 0,20 bis 2,25 Gew.-% und insbesondere bevorzugt 0,25 bis 2,00 Gew.-% mindestens eines Alauns, vorzugsweise eines Kalium-, Natrium-, Ammonium- oder eines Guanidiniumalauns, und
b) 0,001 bis 5,00 Gew.-%, bevorzugt 0,005 bis 4,00 Gew.-%, besonders bevorzugt 0,01 - 2,00 Gew.-%, außerordentlich bevorzugt 0,02 - 1,00 Gew.-% mindestens eines unter der INCI-Bezeichnung Amodimethicone/Morpholinomethyl Silsesquioxane Copolymer bekanntes Silikon enthalten, und
die einen pH-Wert im Bereich von 3,0 bis 4,9, besonders bevorzugt von 3,5 bis 4,8, und insbesondere bevorzugt von 4,0 bis 4,7 aufweisen.

Es hat sich gezeigt, dass die Farbschutz- und Pflegewirkung des kosmetischen Vorbehandlungsmittels weiter verbessert werden kann, wenn bestimmte nichtionische Komponenten ebenfalls in den erfindungsgemäßen Vorbehandlungsmitteln enthalten sind. Zudem haben diese nichtionischen Komponenten positive Effekte auf die Lagerstabilität der erfindungsgemäßen Vorbehandlungsmittel.

Nichtionische Komponenten, die hier besonders geeignet sind, sind Ethoxylate von Decanol, Undecanol, Dodecanol, Tridecanol, Myristylalkohol, Cetylalkohol und/oder Stearylalkohol. Als besonders geeignet haben sich ethoxylierte Tridecanole erwiesen, die mit besonderem Vorzug in die erfindungsgemäßen Vorbehandlungsmittel inkorporiert werden. Besonders bevorzugt sind verzweigte ethoxylierte Tridecanole, insbesondere verzweigte Tridecanole mit 3 bis 5 Ethylenoxid-Einheiten im Molekül.

Erfindungsgemäß besonders bevorzugte Vorbehandlungsmittel enthalten - jeweils bezogen auf ihr Gewicht - 0,001 bis 5 Gew.-%, vorzugsweise 0,005 bis 3,5 Gew.-%, besonders bevorzugt 0,01 bis 2 Gew.-%, weiter bevorzugt 0,05 bis 1 Gew.-% und insbesondere 0,1 bis 0,5 Gew.-% verzweigtes, ethoxyliertes Tridecanol, besonders bevorzugt 0,001 bis 5 Gew.-%, vorzugsweise 0,005 bis 3,5 Gew.-%, besonders bevorzugt 0,01 bis 2 Gew.-%, weiter bevorzugt 0,05 bis 1 Gew.-% und insbesondere 0,1 bis 0,5 Gew.-% verzweigtes, ethoxyliertes Tridecanol mit 3 bis 5 Ethylenoxid-Einheiten im Molekül.

Weitere Tenside und Emulgatoren sind in den erfindungsgemäßen Vorbehandlungsmitteln vorzugsweise nicht oder nur in geringen Mengen enthalten.

Erfindungsgemäß bevorzugte Vorbehandlungsmittel enthalten, bezogen auf das Gesamtgewicht des Mittels, 0,001 bis maximal 6 Gew.-% Tensid(e), wobei die vorgenannten Ethoxylate von Decanol, Undecanol, Dodecanol, Tridecanol, Myristylalkohol, Cetylalkohol und/oder Stearylalkohol mit eingeschlossen sind.

Vorzugsweise werden die erfindungsgemäßen Vorbehandlungsmittel niedrigviskos, das heißt, mit einer Viskosität (bei 20°C gemessen) im Bereich von 10 - 2000 mPas, bevorzugt 20 - 1000 mPas, besonders bevorzugt 50 - 800 mPas, formuliert. Es hat sich darüber hinaus gezeigt, dass verdickende Polymere die erfindungsgemäße Wirkung abschwächen können, so dass bevorzugte erfindungsgemäße Vorbehandlungsmittel dadurch gekennzeichnet sind, dass sie verdickende Polymere in einer Gesamtmenge von ≤ 2,5 Gew.-%, vorzugsweise ≤ 1 Gew.-%, weiter bevorzugt ≤ 0,5 Gew.-% und insbesondere ≤ 0,01 Gew.-%, jeweils bezogen auf das Gewicht des Vorbehandlungsmittels, enthalten.

Die erfindungsgemäßen Vorbehandlungsmittel können weitere Inhaltsstoffe enthalten.

Bevorzugt ist hierbei der Einsatz von mehrwertigen Alkoholen, die Feuchtigkeit spendende Eigenschaften aufweisen. Hier sind erfindungsgemäße Vorbehandlungsmittel bevorzugt, die mindestens einen mehrwertigen Alkohol, bevorzugt ausgewählt aus der Gruppe Sorbit und/oder Glycerin und/oder 1,2-Propylenglycol oder deren Mischungen, in einer Gesamtmenge von 0,05 bis 15 Gew.- %, vorzugsweise 0,1 bis 10 Gew.-%, besonders bevorzugt 0,15 bis 5 Gew.-% und insbesondere 0,15 bis 1 Gew.-%, jeweils bezogen auf das Gewicht des Vorbehandlungsmittels, enthalten.

Für bestimmte Anwendungsbereiche kann es vorteilhaft sein, nur einen der drei oben genannten bevorzugten mehrwertigen Alkohole einzusetzen. In den meisten Fällen ist dabei Glycerin bevorzugt. Allerdings können auf anderen Anwendungsgebieten Mischungen von zwei der drei mehrwertigen Alkohole oder aller drei mehrwertigen Alkohole bevorzugt sein.

Neben Sorbit, Glycerin und 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)-bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800, eingesetzt werden. Besonders bevorzugt ist der Einsatz von Glycerin, so dass Mittel, die außer Glycerin keine anderen mehrwertigen Alkohole enthalten, besonders bevorzugt sind.

Im Hinblick auf die Vorbehandlung vor einer oxidativen Färbe- und/oder Bleichbehandlung ist der Einsatz bestimmter Pflegestoffe in den Vorbehandlungsmitteln bevorzugt.

Erfindungsgemäß bevorzugte Vorbehandlungsmittel sind dadurch gekennzeichnet, dass sie zusätzlich Pflegestoff(e) in einer Gesamtmenge von 0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-%, jeweils bezogen auf das Gesamtgewicht des Vorbehandlungsmittels, enthalten. Bevorzugte Pflegestoff(e) sind ausgewählt aus mindestens einer der nachfolgend genannten Gruppen:
i. L-Carnitin und/oder seiner Salze;
ii. Taurin und/oder seiner Salze;
iii. Niacinamid;
iv. Ubichinon;
v. Ectoin;
vi. Vitamine und/oder Provitamine und/oder Vitaminderivate;
vii. Flavonoide.

Als weiteren Inhaltsstoff können die erfindungsgemäßen Vorbehandlungsmittel mit besonderem Vorzug eine oder mehrere Aminosäuren enthalten.

Erfindungsgemäß besonders bevorzugte Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, *S*-Methyl-L-methionin, *S*-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Vorbehandlungsmittel enthalten eine oder mehrere Aminosäuren in engeren Mengenbereichen. Hier sind erfindungsgemäße Vorbehandlungsmittel dadurch gekennzeichnet, dass sie als Pflegestoff 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), vorzugsweise aus der Gruppe Glycin und/oder Alanin und/oder Valin und/oder Lysin und/oder Leucin und/oder Threonin enthalten, jeweils bezogen auf das Gesamtgewicht des Vorbehandlungsmittels.

Ein dritter Gegenstand der vorliegenden Erfindung ist die Verwendung eines kosmetischen Mittels, das - bezogen auf sein Gewicht -
a) 0,01 bis 4,00 Gew.-% mindestens eines Alauns und
b) 0,001 bis 5,00 Gew.-% mindestens eines Silikons enthält, und
das einen pH-Wert im Bereich von 2,5 bis 5 aufweist,
zum Farberhalt gefärbter keratinischer Fasern.

Für bevorzugte Ausführungsformen der erfindungsgemäßen Verwendung gilt mutatis mutandis das zu dem erfindungsgemäßen Verfahren und das zu dem erfindungsgemäßen kosmetischen Mittel Gesagte.

### Beispiele

Haarsträhnen wurden eine Minute lang in eine wässrige Zusammensetzung eingetaucht, die
- 2,00 Gew.-% KAl(SO₄)₂ x 12 H₂O,
- 0,05 Gew.-% Amodimethicone/Morpholinomethyl Silsesquioxane Copolymer,
- fakultativ 0,001 bis 1,0 Gew.-% Glycerin und/oder 0,005 Gew.-% bis 1,0 Gew.-% verzweigtes Trideceth-5, und
- Wasser (ad 100) enthielt.

Anschließend wurden die Haarsträhnen getrocknet, eine frisch zubereitete Färbecreme-Oxidationsmittelmischung auf die Strähnen aufgetragen und 30 Minuten einwirken gelassen. Das Färbemittel wurde anschließend mit Wasser ausgespült und die Haare gekämmt und ggfs. getrocknet.

Durch die erfindungsgemäße Vorbehandlung mit wässrigen Zusammensetzungen, die mindestens ein Alaun und mindestens eine unter der INCI-Bezeichnung Amodimethicone/Morpholinomethyl Silsesquioxane Copolymer bekannte Verbindung enthalten, konnte ein ausgezeichneter Farbschutz erzielt werden. Insbesondere konnte die Farbtreue der Färbung auf der sogenannten b-Achse im lab-Farbraum (EN ISO 11664-4) gestärkt werden (geringere Verschiebung der Farbe in Richtung gelb bzw. blau).

Darüber hinaus konnte die Haarschädigung gegenüber einer oxidativen Färbung ohne erfindungsgemäße Vorbehandlung deutlich verringert werden.

Auch die sensorische Beurteilung des Haars durch geschulte Friseure belegte, dass sich erfindungsgemäß vorbehandelte Haare nach der Färbung weicher und glatter anfühlen als nicht vorbehandelte Haare nach der Färbung.

## Patentansprüche

1. Kosmetisches Verfahren zum Farberhalt gefärbter keratinischer Fasern, bei dem
i. eine kosmetische Zusammensetzung als Vorbehandlungsmittel auf keratinische Fasern aufgebracht wird, und
ii. die keratinischen Fasern innerhalb eines Zeitraums von 5 Sekunden bis 24 Stunden nach Schritt i. einer oxidativen Färbung unterzogen werden,
**dadurch gekennzeichnet, dass** die kosmetische Zusammensetzung - bezogen auf ihr Gewicht - 0,01 bis 4,00 Gew.-% mindestens eines Alauns enthält und einen pH-Wert im Bereich von 2,5 bis 5 aufweist.

2. Verfahren nach einem der vorhergehenden Ansprüche, **dadurch gekennzeichnet, dass** die kosmetische Zusammensetzung ein Alaun der Formel M^{I}Al(SO₄)₂ x 12 H₂O enthält, in der M' für ein Kalium- Natrium-, Ammonium- oder ein Guanidiniumion steht.

3. Verfahren nach einem der vorhergehenden Ansprüche, **dadurch gekennzeichnet, dass** die kosmetische Zusammensetzung - bezogen auf ihr Gewicht - 0,05 bis 3,50 Gew.-%, bevorzugt 0,10 bis 3,00 Gew.-%, mehr bevorzugt 0,15 bis 2,50 Gew.-%, besonders bevorzugt 0,20 bis 2,00 Gew.-% und insbesondere bevorzugt 0,25 bis 1,50 Gew.-% mindestens eines Kalium-, Natrium-, Ammonium- und/oder Guanidiniumalauns enthält.

4. Verfahren nach einem der vorhergehenden Ansprüche, **dadurch gekennzeichnet, dass** die kosmetische Zusammensetzung einen pH-Wert von 3,0 bis 4,9, besonders bevorzugt von 3,5 bis 4,8, und insbesondere bevorzugt von 4,0 bis 4,7 aufweist.

5. Verfahren nach einem der vorhergehenden Ansprüche, **dadurch gekennzeichnet, dass** die kosmetische Zusammensetzung - bezogen auf ihr Gewicht - zusätzlich 0,001 bis 5,00 Gew.-% mindestens eines Silikons enthält.

6. Verfahren nach Anspruch 5, **dadurch gekennzeichnet, dass** als Silikon mindestens eine unter der INCI-Bezeichnung Amodimethicone/Morpholinomethyl Silsesquioxane Copolymer bekannte Verbindung eingesetzt wird.

7. Kosmetisches Mittel zur Vorbehandlung keratinischer Fasern vor einer (vorzugsweise oxidativen) Färbung und/oder Aufhellung, **dadurch gekennzeichnet, dass** es - bezogen auf sein Gewicht -
a) 0,01 bis 4,00 Gew.-% mindestens eines Alauns und
b) 0,001 bis 5,00 Gew.-% mindestens eines Silikons enthält, und
einen pH-Wert im Bereich von 2,5 bis 5 aufweist.

8. Kosmetisches Mittel nach Anspruch 7, **dadurch gekennzeichnet, dass** es - bezogen auf sein Gewicht - 75,00 bis 99,98 Gew.-%, mehr bevorzugt 80,00 bis 99,50 Gew.-%, besonders bevorzugt 85,00 bis 99,00 Gew.-% und insbesondere 90,00 bis 98,50 Gew.-% Wasser enthält.

9. Verwendung eines kosmetischen Mittels, das - bezogen auf sein Gewicht -
a) 0,01 bis 4,00 Gew.-% mindestens eines Alauns und
b) 0,001 bis 5,00 Gew.-% mindestens eines Silikons enthält, und
das einen pH-Wert im Bereich von 2,5 bis 5 aufweist,
zum Farberhalt gefärbter keratinischer Fasern.

## Claims

1. A cosmetic method for the color retention of colored keratin fibers, in which
i. a cosmetic composition is applied to keratin fibers as a pretreatment agent, and
ii. after step i., the keratin fibers are subjected to oxidative coloring within a period of from 5 seconds to 24 hours,
**characterized in that** the cosmetic composition contains, based on its weight, 0.01 to 4.00 wt.% of at least one alum and has a pH in the range of from 2.5 to 5.

2. The method according to one of the preceding claims, **characterized in that** the cosmetic composition contains an alum of formula M^{I}Al(SO₄)₂ x 12 H₂O, in which M^{I} represents a potassium ion, sodium ion, ammonium ion or a guanidinium ion.

3. The method according to one of the preceding claims, **characterized in that** the cosmetic composition contains, based on its weight, 0.05 to 3.50 wt.%, preferably 0.10 to 3.00 wt.%, more preferably 0.15 to 2.50 wt.%, particularly preferably 0.20 to 2.00 wt.% and more particularly preferably 0.25 to 1.50 wt.% of at least one potassium alum, sodium alum, ammonium alum and/or guanidinium alum.

4. The method according to one of the preceding claims, **characterized in that** the cosmetic composition has a pH of from 3.0 to 4.9, particularly preferably from 3.5 to 4.8 and more particularly preferably from 4.0 to 4.7.

5. The method according to one of the preceding claims, **characterized in that** the cosmetic composition additionally contains, based on its weight, 0.001 to 5.00 wt.% of at least one silicone.

6. The method according to claim 5, **characterized in that** at least one compound known under the INCI name Amodimethicone/Morpholinomethyl Silsesquioxane Copolymer is used as the silicone.

7. A cosmetic agent for pretreating keratin fibers prior to (preferably oxidative) coloring and/or lightening, **characterized in that** it contains, based on its weight,
a) 0.01 to 4.00 wt.% of at least one alum and
b) 0.001 to 5.00 wt.% of at least one silicone, and
has a pH in the range of from 2.5 to 5.

8. The cosmetic agent according to claim 7, **characterized in that** it contains, based on its weight, 75.00 to 99.98 wt.%, more preferably 80.00 to 99.50 wt.%, particularly preferably 85.00 to 99.00 wt.% and in particular 90.00 to 98.50 wt.%, water.

9. The use of a cosmetic agent that contains, based on its weight,
a) 0.01 to 4.00 wt.% of at least one alum and
b) 0.001 to 5.00 wt.% of at least one silicone, and
that has a pH in the range of from 2.5 to 5,
for the color retention of colored keratin fibers.

## Revendications

1. Procédé cosmétique permettant la tenue de la couleur de fibres kératiniques colorées, dans lequel
i. une composition cosmétique est appliquée en tant qu'agent de prétraitement sur les fibres kératiniques, et
ii. les fibres kératiniques sont soumises à une coloration oxydative pour une durée de 5 secondes à 24 heures selon l'étape i.,
**caractérisé en ce que** la composition cosmétique - par rapport à son poids - contient de 0,01 à 4,00 % en poids d'au moins un alun et présente un pH dans la gamme de 2,5 à 5.

2. Procédé selon l'une des revendications précédentes, **caractérisé en ce que** la composition cosmétique contient un alun de formule M'Al(SO₄)₂ x 12 H₂O, dans laquelle M' représente un ion potassium, sodium, ammonium ou guanidinium.

3. Procédé selon l'une des revendications précédentes, **caractérisé en ce que** la composition cosmétique - par rapport à son poids - contient de 0,05 à 3,50 % en poids, de préférence de 0,10 à 3,00 % en poids, de manière davantage préférée de 0,15 à 2,50 % en poids, de manière particulièrement préférée de 0,20 à 2,00 % en poids et en particulier de préférence de 0,25 à 1,50% en poids d'au moins un alun de potassium, de sodium, d'ammonium et/ou de guanidinium.

4. Procédé selon l'une des revendications précédentes, **caractérisé en ce que** la composition cosmétique présente un pH de 3,0 à 4,9, de manière particulièrement préférée de 3,5 à 4,8 et en particulier de préférence de 4,0 à 4,7.

5. Procédé selon l'une des revendications précédentes, **caractérisé en ce que** la composition cosmétique - par rapport à son poids - contient en outre de 0,001 à 5,00 % en poids d'au moins une silicone.

6. Procédé selon la revendication 5, **caractérisé en ce qu'**au moins un composé connu sous le nom INCI Amodimethicone/Morpholinomethyl Silsesquioxane Copolymer est utilisé en tant que silicone.

7. Agent cosmétique de prétraitement de fibres kératiniques avant une coloration et/ou un éclaircissement (de préférence oxydatif), **caractérisé en ce qu'**il contient - par rapport à son poids -
a) de 0,01 à 4,00 % en poids d'au moins un alun et
b) de 0,001 à 5,00% en poids d'au moins une silicone, et
présente un pH compris entre 2,5 et 5.

8. Composition cosmétique selon la revendication 7, **caractérisée en ce qu'**elle contient - par rapport à son poids - de 75,00 à 99,98 % en poids, de manière davantage préférée de 80,00 à 99,50 % en poids, de manière particulièrement préférée de 85,00 à 99,00 % en poids et en particulier de 90,00 à 98,50 % en poids d'eau.

9. Utilisation d'un agent cosmétique qui contient - par rapport à son poids -
a) de 0,01 à 4,00 % en poids d'au moins un alun et
b) de 0,001 à 5,00 % en poids d'au moins une silicone, et
qui présente un pH compris entre 2,5 et 5,
pour la tenue de la couleur de fibres kératiniques colorées.
